# EUROPÄISCHE PATENTANMELDUNG

(11) **EP 0 702 021 A1**
(43) Veröffentlichungstag der Anmeldung: **20.03.1996**
(21) Anmeldenummer: 95110917.2
(22) Anmeldetag: 12.07.1995
(51) Int. Cl.: C07H 15/04

(54) **Verfahren zur Herstellung von Alkyloligoglycosiden**

(30) Priorität: 07.09.1994 DE 4431853
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Herstellung von Alkyloligoglycosiden durch Umglycosidierung von C₂- bis C₆-Alkylglycosiden mit C₈- bis C₂₀-Fettalkoholen wird dadurch verbessert, daß man den Fettalkohol auf mindestens 80 °C vorwärmt, ihn mit dem Alkylglycosid mischt, dann die Reaktion unter einem Druck von 1,1 bis 10 bar vornimmt und nach der Reaktion C₂- bis C₆-Alkohol unter Vakuum abflasht.
Man erhält farblich gute Produkte. Zu Verkrustungen und Vercrackungen in den Apparaturen kommt es nicht.

## Beschreibung

Die Erfindung betrifft ein neues Verfahren zur Herstellung von Alkyloligoglycosiden, die C₈- bis C₂₀-Alkylketten aufweisen, durch säurekatalysierte Umglycosidierung von C₂- bis C₆-Alkylglycosiden mit C₈- bis C₂₀-Fettalkoholen.

Alkyloligoglycoside mit C₈- bis C₂₀-Alkylgruppen können ganz oder teilweise aus nachwachsenden Rohstoffen hergestellt werden. Sie gewinnen deshalb und auch wegen ihrer sehr guten biologischen Abbaubarkeit zunehmend an Bedeutung. Die Produkte haben neben ihren interessanten Tensideigenschaften den Vorteil, daß man ihre Polarität über die Länge der Alkylkette und über den Glycosidierungsgrad genau einstellen kann. Dadurch können die Alkyloligoglycoside auf ihr Anwendungsgebiet gezielt ausgerichtet werden.

Bei der zweistufigen Alkyloligoglycosidherstellung wird zunächst eine Glycosidierung durchgeführt. Aus Sacchariden und C₂- bis C₆-Alkoholen werden dabei Alkylglycoside mit kurzkettigen Alkylgruppen hergestellt. Diese Produkte werden dann in der zweiten Stufe durch Umglycosidierung mit C₈- bis C₂₀-Alkoholen in die gewünschten Alkyloligoglycoside mit Tensideigenschaften übergeführt.

Dieser Weg der Herstellung ist lange bekannt. Neuere Anmeldungen auf diesem Gebiet befassen sich häufig mit der Herstellung farblich verbesserter Produkte. Dabei werden zum Teil Reduktionsmittel zugesetzt oder spezielle Apparaturen verwendet.

Meist erfolgt die Umglycosidierung jedoch in einem von außen beheizten Rührkessel oder in einer beheizten Rührkesselkaskade.

Nach EP-A-0 514 628 kann die Umglycosidierung auch in einem Verdampfer vorgenommen werden. Nach EP-A-0 514 627 wird die Umglycosidierung in einer Reaktionskolonne durchgeführt. Dabei stellt man ein Molverhältnis von Alkylglycosid mit kurzem Alkylrest zu langkettigem Alkohol von 1 : 2 bis 1 : 15 ein und erhält Alkyloligoglycoside mit Glycosidierungsgraden von vorzugsweise 1,2 bis 3.

WO 93/101 33 beschreibt ein zweistufiges Verfahren zur Herstellung von Alkyloligoglycosiden, das kontinuierlich oder diskontinuierlich durchgeführt werden kann und bei dem ein Glucosesirup mit einem Anteil an monomerer Glucose von 90 bis 100 % eingesetzt wird. Das hier beschriebene Verfahren ist insgesamt recht aufwendig. Es erfordert sowohl bei der Glycosidierung als auch bei der Umglycosidierung eine Nachreaktion.

Bei der Umglycosidierung wird hier der auf die Reaktionstemperatur erhitzte Fettalkohol in einem Rührkessel mit Mantelheizung vorgelegt. Das kurzkettige Alkylglycosid wird zudosiert. Während der Umglycosidierung wird dann die Mischung bei leichtem Vakuum von etwa 100 mbar in einen Verdampfer geleitet.

In den genannten Verfahren wird die Wärme für die Reaktionstemperatur von außen über den Mantel des Reaktors eingeführt.

Diese Verfahrensweise hat aber den Nachteil, daß die Wandtemperatur im allgemeinen mindestens 5 bis 10 °C oberhalb der Reaktionstemperatur liegt. Dadurch können empfindliche Produkte an der Reaktorwand thermisch geschädigt und verfärbt werden. Es kann deshalb zu Verkrustungen und Verstopfungen in den Apparaturen und Rohrleitungen kommen.

Aufgabe der vorliegenden Erfindung war es deshalb, bei der Umglycosidierung Überhitzungen insbesondere durch erhöhte Manteltemperaturen zu vermeiden.

Die Aufgabe wird erfindungsgemäß dadurch gelöst, daß man den Fettalkohol auf mindestens 80 °C vorwärmt, ihn mit dem C₂- bis C₆-Alkylglycosid mischt und bei einem Druck von 1,1 bis 10 bar umsetzt und nach der Reaktion C₂- bis C₆-Alkohol unter Vakuum abflasht.

Vorzugsweise wird der Fettalkohol dabei auf 80 bis 150 °C und ganz besonders bevorzugt auf 100 bis 130 °C vorgewärmt. Die Reaktionstemperatur bei der Umglycosidierung liegt meist bei 60 bis 140 °C und im besonderen bei 80 bis 120 °C. Vorzugsweise wird dabei ein Druck von 1,3 bis 5 bar eingestellt.

Das Abflashen, das spontane Entspannen der Reaktionsmischung, erfolgt im allgemeinen hinter einer Drosselstelle (Ventil, Düse usw.). Dazu wird außen vorzugsweise ein Vakuum von 1 bis 700 mbar angelegt. Im besonderen beträgt der Absolutdruck dabei 3 bis 40 mbar.

Bei der Herstellung der C₂- bis C₆-Alkylglycoside setzt man als Monosaccharid beispielsweise Glucose, Mannose, Galaktose, Gulose, Allose oder Talose ein. Vorzugsweise geht man dabei jedoch von Glucose aus. Der Alkylteil leitet sich beispielsweise von den Alkoholen Ethanol, n-Propanol, Isopropanol, n-Butanol, t-Butanol, Amylalkohol oder Hexanol her. Hier wird n-Butanol bevorzugt, so daß für die Umglycosidierung vorzugsweise n-Butylglucosid eingesetzt wird.

Die C₂- bis C₆-Alkylglycoside, die in geringem Umfange auch Oligomere enthalten können, werden vorzugsweise gelöst im zugehörigen Alkohol für die Umglycosidierung eingesetzt. 25- bis 60%ige Alkylglycosid-Lösungen werden dabei bevorzugt verwendet.

Beim Abflashen wird deshalb nicht nur der bei der Umglycosidierung gebildete Alkohol sondern auch der Alkohol, der als Lösemittel gedient hatte, verdampft.

Die C₈- bis C₂₀-Fettalkohole können linear oder verzweigt sein. Sie können auch olefinische Doppelbindungen enthalten. Man kann natürliche oder synthetische Fettalkohole oder Fettalkoholgemische einsetzen. Als Beispiele seien Octanol, Decanol, 10-Undecen-1-ol, Dodecanol, Myristylalkohol und Stearylalkohol genannt. Vorzugsweise werden Fettalkohole mit 10 bis 14 C-Atome eingesetzt. Meist wird das Alkylglycosid mit C₂- bis C₆-Alkylgruppen im Molverhältnis von 1 : 4 bis 1 : 10 mit dem Fettalkohol umgesetzt.

Bei der Umglycosidierung kann man als saure Katalysatoren Mineralsäuren, wie Schwefel- oder Salzsäure, einsetzen. Gut geeignet sind auch organische Säuren, wie beispielsweise Aryl-, Alkyl- oder Aralkylsulfonsäuren. Dabei wird der Katalysator vorzugsweise gelöst oder suspendiert im vorgewärmten Fettalkohol in die Reaktionsmischung eingebracht.

Die Reaktion kann mit Vorteilen in einem Mischer vorgenommen werden. Dabei kann man den vorgewärmten Fettalkohol einschließlich des Katalysators und gleichzeitig eine C₂- bis C₆-Alkylglycosid-Lösung in den Mischer eindosieren. Man kann die Alkylglycosid-Lösung auch vorlegen und Fettalkohol und Katalysator zudosieren. Die Umglycosidierung kann aber auch in einem Rührkessel, in einer Rührkesselkaskade oder nach vorheriger Mischung in einem Rohrreaktor durchgeführt werden.

Die erfindungsgemäß hergestellten Alkyloligoglycoside weisen meist Glycosidierungsgrade von 1 bis 3 auf. Danach werden hier auch Produkte mit dem Glycosidierungsgrad 1 als Alkyloligoglycoside bezeichnet. Der Glycosidierungsgrad liegt vorzugsweise im Bereich von 1,1 bis 1,5.

Das Produkt der Umglycosidierung wird in bekannter Art und Weise mit geeigneten Basen neutralisiert. Der überschüssige Fettalkohol wird dann destillativ abgetrennt, was in einem Dünnschicht- oder Kurzwegverdampfer erfolgen kann. Das gewonnene Alkyloligoglycosid wird dann mit Wasser abgemischt und mit Peroxid gebleicht. Das Endprodukt weist eine Iodfarbzahl (IFZ) von unter 20 und vorzugsweise einen Restfettalkoholgehalt von unter 1 % auf.

Das erfindungsgemäße Verfahren der direkten Wärmezufuhr ermöglicht es, daß die Wandtemperatur des Reaktors die Produkttemperatur der Reaktionsmischung nicht übersteigt. Es kommt daher nicht zu Überhitzungen an den Reaktorwänden. Das Verfahren führt zu Reaktionsmischungen mit einem sehr geringen Anteil an ungelösten Bestandteilen. Die Farbqualität der Produkte wird verbessert. Es kommt nicht zu Vercrackungen und Verkrustungen. Rohrleitungen und Reaktoren neigen deshalb nicht zu Verstopfungen. Das erhöht die Betriebssicherheit.

Das folgende Beispiel soll die Erfindung verdeutlichen.

### Beispiel 1

In einem Wärmetauscher (Fläche: 0,4 m²) werden stündlich 40 kg C₁₂/C₁₄-Fettalkohol und 0,2 kg butanolische p-Toluolsulfonsäurelösung (173 g/l p-Toluolsulfonsäure) von 40 auf 120 °C aufgeheizt. Anschließend wird eine 35%ige Butylglucosid-Lösung zugeführt, worauf die Flüssigkeiten in einem Static-Mischer homogen gemischt werden. Nach dem Durchströmen einer Reaktionsstrecke (Verweilzeit 10 Minuten, Druck 1,5 bar) wird Butanol hinter einem Regelventil bei einem Vakuum von 10 mbar abgeflasht.

Nach Neutralisation und Destillation des Fettalkohols im Dünnschichtverdampfer und Bleichung mit H₂O₂ in wäßriger Lösung wird ein Produkt mit einer IFZ (einer 50%igen wäßrigen Lösung) von 10 erhalten.

## Patentansprüche

1. Verfahren zur Herstellung von Alkyloligoglycosiden, die C₈- bis C₂₀-Alkylreste aufweisen, durch säurekatalysierte Umglycosidierung von C₂- bis C₆-Alkylglycosiden mit C₈- bis C₂₀-Fettalkoholen,
dadurch gekennzeichnet, daß
- man den Fettalkohol auf mindestens 80 °C vorwärmt,
- ihn mit dem Alkylglycosid mischt und unter einem Druck von 1,1 bis 10 bar umsetzt
- und nach der Reaktion C₂- bis C₆-Alkohol unter Vakuum abflasht.

2. Verfahren nach Anspruch 1,
dadurch gekennzeichnet,
daß der Fettalkohol auf 80 bis 150 °C, vorzugsweise auf 100 bis 130 °C, vorgewärmt wird.

3. Verfahren nach Anspruch 1,
dadurch gekennzeichnet,
daß die Reaktionstemperatur 60 bis 140 °C, vorzugsweise 80 bis 120 °C, beträgt.

4. Verfahren nach Anspruch 1,
dadurch gekennzeichnet,
daß bei der Reaktion ein Druck von 1,3 bis 5 bar eingestellt wird.

5. Verfahren nach Anspruch 1,
dadurch gekennzeichnet,
daß der C₂- bis C₆-Alkohol bei 1 bis 700 mbar, vorzugsweise bei 3 bis 40 mbar, abgeflasht wird.

6. Verfahren nach Anspruch 1,
dadurch gekennzeichnet,
daß man den sauren Katalysator gelöst oder suspendiert im vorgewärmten Fettalkohol in die Reaktionsmischung einführt.
